# EUROPEAN PATENT SPECIFICATION

(11) **EP 2 103 552 B1**
(45) Date of publication and mention of the grant of the patent: **19.10.2011**
(21) Application number: 09250789.6
(22) Date of filing: 20.03.2009
(51) Int. Cl.: B65D 85/36

(54) **Soft contact lens package product and method of packaging soft contact lens**
Weiches Kontaktlinsenverpackungsprodukt und Verfahren zur Verpackung einer weichen Kontaktlinse
Produit de conditionnement de lentilles de contact souples et procédé pour le conditionnement de lentilles de contact souples

(30) Priority: 21.03.2008 JP 2008073084
(43) Date of publication of application: 23.09.2009
(73) Proprietor: Menicon Co., Ltd., Nagoya-shi, Aichi 460-0006 (JP)
(72) Inventor: Yamada, Yoshitaka, Kasugai-shi Aichi-ken, 487-0032 (JP); Yamamoto, Takashi, Kasugai-shi Aichi-ken, 487-0032 (JP); Yamaguchi, Hiroyuki, Nagoya-shi Aichi-ken, 460-0006 (JP)
(74) Representative: Paget, Hugh Charles Edward

(56) References cited:
- EP-A- 1 752 058
- US-A1- 2005 260 098
- US-A1- 2007 104 744
- US-A1- 2007 286 767

## Description

### BACKGROUND OF THE INVENTION

### Field of the Invention

The present invention relates to a soft contact lens package product and a method of packaging a soft contact lens, particularly to a soft contact lens package product suitable for shipping a hydrated soft contact lens and a method of packaging such a soft contact lens, and further to a useful packaging solution for a hydrated soft contact lens, an effective method of treating a hydrated soft contact lens and a sustained-release soft contact lens having excellent characteristics.

### Discussion of Related Art

Hydrated soft contact lenses (SCLs) have hitherto been shipped as soft contact lens package products (SCL package products) from manufacturers in a state where they are immersed in liquid media such as saline and housed in predetermined sealed containers together with the liquid media, in order to provide them to wearers as consumers (users) thereof (see JP-T-2004-523777 (patent document 1) and JP-A-2007-44536 (patent document 2), for example).

Further, as a liquid medium which is used for storage or transportation of a SCL and in which such a SCL is immersed, for example, JP-A-10-130448 (patent document 3) discloses that an aqueous solution containing polyvinyl alcohol having an average polymerization degree of less than 1,000 and a saponification degree of 85 to 95 mol% at a concentration of 0.1 to 3.0 g/100 ml is used in place of conventional saline. It is said that use of the aqueous solution containing polyvinyl alcohol enhances wettability of the SCL, which results in improvement of wearing feeling.

However, in the SCL immersed in the aqueous solution containing a polymer compound such as polyvinyl alcohol, adhesion of the polymer compound to the SCL is generally realized by adsorption of a hydrophobic portion of the polymer composition to a hydrophobic portion of a SCL surface by weak van der Waals force. For this reason, when such a SCL is worn on the eye, the polymer compound adhered to the SCL is easily washed away with the tear fluid. Accordingly, there may cause a problem of failing to continuously obtain advantages such as improvement in wearing feeling due to active ingredients comprising such polymer compounds.

Moreover, the polymer compound such as polyvinyl alcohol as described above can scarcely enter a matrix (tissue) of the SCL as it is, because of its giant molecule, and is adsorbed only by a surface thereof. Accordingly, there may also cause a problem that the adsorption thereof is quantitatively insufficient. To solve the problem, it is considered that the SCL is immersed in a highly-concentrated (highly-viscous) aqueous solution containing the polymer compound such as polyvinyl alcohol to adhere a large amount of the polymer compound to the SCL surface. However, when such a SCL to which a large amount of the polymer compound is adhered is worn on the eye, the presence of a polymer compound layer formed on the SCL surface causes blurred vision, resulting in failure to secure good visibility. Accordingly this has been unfavorable.

Meanwhile, JP-A-8-157886 (patent document 4) discloses, as a contact lens solution intended to improve wearing feeling of a SCL, a contact lens solution in which one or two or more compounds selected from the group consisting of aminoethylsulfonic acid, K L-aspartate and Mg L-aspartate is incorporated. Further, JP-A-2000-155295 (patent document 5) discloses a contact lens care agent comprising trimethylglycine.

When low-molecular compounds such as aminoethylsulfonic acid and trimethylglycine are used as ingredients for improving wearing feeling of the SCL, it is considered that active ingredients comprising those low-molecular compounds can enter not only the surface of the SCL, but also the matrix of the SCL at larger ratios.

However, the compound such as aminoethylsulfonic acid cannot sufficiently exert adsorbability by van der Waals force because of its low molecular weight, and is thought to be in a state where it is only dispersed around the SCL or in the matrix thereof, in an aqueous solution. For this reason, also in the SCL immersed in such a aqueous solution containing low-molecular compound, when it is worn on the eye, such a low-molecular compound adhered to the SCL is easily washed away with the tear fluid in a short period of time. Accordingly, there has been inherent a problem of failing to continuously obtain advantages of active ingredients comprising such low-molecular compounds.

As described above, in all of the active ingredients comprising the polymer compounds such as polyvinyl alcohol and the active ingredients comprising the low-molecular compounds such as aminoethylsulfonic acid, there has been inherent the problem that those active ingredients are easily washed away with the tear fluid when the SCL is worn on the eye, resulting in failure to continuously obtain the desired advantages of such active ingredients. Further, such a problem is also arisen not only in the above-exemplified ingredients for improving wearing feeling of the SCL, but also in anti-inflammatory ingredients for reducing inflammation and various other ingredients. There is inherent the problem that the retention (residence) time of those active ingredients on the SCL is short, so that the duration of effect is insufficient.

US 2005/0260098 describes compositions for use in contact lens care solutions. The compositions contain one or more oligosaccharides, which may be cationic, anionic, non-ionic or a combination thereof. Stearyl dihydroxypropyldimonium oligosaccharide (SDO) is mentioned as being preferred.

US 2007/0104744 describes solutions for contact lens care, which employ select B vitamins; pyridoxine and its salts; and thiamine and its salts in order to more effectively preserve the solutions and to reduce the degree to which cationic preservatives will deposit on contact lenses.

### SUMMARY OF THE INVENTION

The present invention has been made in the light of the situations described above. It is therefore an object of the invention to provide a soft contact lens package product which can be advantageously provided to hydrated soft contact lens wearers (users). It is another object to provide a method of shipping a soft contact lens shipped in the form of such a soft contact lens package product, and a method of packaging a soft contact lens by which such a soft contact lens package product can be advantageously obtained.

Further, it is still another object to provide a packaging solution for a soft contact lens, which is advantageously used for obtaining a hydrated soft contact lens in which advantages of predetermined active ingredients can be continuously exerted over a long period of time, a method of treating a hydrated soft contact lens by which such a soft contact lens can be advantageously obtained, and a sustained-release soft contact lens having such excellent characteristics.

In order to solve the above-mentioned problems or problems grasped from a description of the entire specification or drawings, the invention can be advantageously carried out in various modes as described below, and the respective modes described below can also be employed in any combination. It is to be understood that the modes or technical features of the invention can be recognizable based on the inventive concept disclosed in the description of the entire specification and the drawings without being limited to the details of the following description.

(1) A soft contact lens package product, comprising: a liquid medium containing at least one anionic compound having two or more anionic functional groups in one molecule and at least one cationic compound having two or more cationic functional groups in one molecule; at least one hydrated soft contact lens immersed in the liquid medium; and a sealable container in which the liquid medium and the at least one hydrated soft contact lens are housed, the sealable container being sealed so that the inside thereof being held in a sterile condition.

(2) The soft contact lens package product according to the above mode (1), wherein the anionic compound has at least one of a carboxyl group, a sulfo group and a phosphoric acid group as the anionic functional group.

(3) The soft contact lens package product according to the above mode (1) or (2), wherein the anionic compound is selected from the group consisting of an anionic polysaccharide, glycyrrhizic acid, a salt of the glycyrrhizic acid, a purine base nucleotide and a peptide.

(4) The soft contact lens package product according to the above mode (3), wherein the anionic polysaccharide is selected from the group consisting of chondroitin sulfuric acid, a salt of the chondroitin sulfuric acid, hyaluronic acid, a salt of the hyaluronic acid, alginic acid and a salt of the alginic acid.

(5) The soft contact lens package product according to any one of the above modes (1) to (4), wherein the cationic compound has at least one of an amino group, an ammonium group and a biguanide group as the cationic functional group.

(6) The soft contact lens package product according to any one of the above modes (1) to (5), wherein the cationic compound is selected from the group consisting of a vitamin, a purine base, a nucleoside derived from the purine base, chlorpheniramine, a salt of the chlorpheniramine, and allantoin.

(7) The soft contact lens package product according to the above mode (6), wherein the vitamin is selected from the group consisting of vitamin B₁ and vitamin B₁₂.

(8) The soft contact lens package product according to the above mode (6) or (7), wherein the purine base is one of adenine and guanine, and the nucleoside derived from the purine is one of adenosine and guanosine.

(9) A method of shipping a soft contact lens, comprising the step of shipping at least one hydrated soft contact lens in the form of the soft contact lens package product according to any one of the above modes (1) to (8), thereby providing the at least one hydrated soft contact lens to a wearer.

(10) A method of packaging a soft contact lens, comprising the steps of: (a) providing a sealable container having a size sufficient to house at least one hydrated soft contact lens; (b) providing a liquid medium to be housed in the container, the liquid medium containing at least one anionic compound having two or more anionic functional groups in one molecule and at least one cationic compound having two or more cationic functional groups in one molecule; (c) housing the liquid medium in the container together with the at least one hydrated soft contact lens; and (d) sealing the container in a state where the at least one hydrated contact lens is immersed in the liquid medium.

(11) The method of packaging a soft contact lens according to the above mode (10), further comprising the step of heat treating the sealed container.

(12) A packaging solution for a hydrated soft contact lens, comprising a liquid medium containing at least one anionic compound having two or more anionic functional groups in one molecule and at least one cationic compound having two or more cationic functional groups in one molecule.

(13) A method of treating a soft contact lens, comprising the step of immersing a hydrated soft contact lens in a liquid medium containing at least one anionic compound having two or more anionic functional groups in one molecule and at least one cationic compound having two or more cationic functional groups in one molecule, the at least one anionic compound and the at least one cationic compound being allowed to enter a matrix of the hydrated soft contact lens and be ion bonded in the matrix to constitute pseudo macromolecules.

(14) The method of treating a soft contact lens according to the above mode (13), wherein the liquid medium in which the hydrated soft contact lens is immersed is heated.

(15) A sustained-release soft contact lens in which at least one anionic compound having two or more anionic functional groups in one molecule and at least one cationic compound having two or more cationic functional groups in one molecule are allowed to enter a matrix of a hydrated soft contact lens and are ion bonded in the matrix thereof to constitute pseudo macromolecules, the compounds being gradually released from the macromolecules to the tear fluid when the contact lens is worn on an eye.

As described above, the soft contact lens package product according to the invention is provided and distributed to the market in the form in which at least one hydrated soft contact lens (SCL) is immersed in a liquid medium containing at least one anionic compound having two or more anionic functional groups in one molecule and at least one cationic compound having two or more cationic functional groups in one molecule, and sealed in a predetermined container. Accordingly, in the course of distribution over a long period of time, as well as being ion bonded on a surface of the SCL, the anionic compound and the cationic compound sealed in the container enter the inside of a matrix of the SCL to be ion bonded therein. Consequently, pseudo macromolecules are constructed by ionic interaction both inside and outside the matrix tissue of the SCL, independently of a hydrated polymer constituting the SCL. Further, in the invention, the viscosity of the liquid medium can be effectively increased by such pseudo macromolecules, which makes it possible to advantageously improve wearing feeling of the SCL.

In particular, such pseudo macromolecules are formed therein by ion bond of the anionic compound and the cationic compound, which have relatively strong bonding force based on ionic interaction. Moreover, in the SCL, such macromolecules are formed while free migration thereof is restrained by the matrix tissue constituting the SCL. Accordingly, when the SCL in which such macromolecules are formed is worn on the eye, such macromolecules are advantageously prevented from being immediately washed away with the tear fluid. Thus, the advantage of improved wearing feeling by such pseudo macromolecules can be continuously maintained over a long period of time.

Further, when the SCL taken out of the SCL package product according to the invention is worn on the eye, the anionic compound and the cationic compound in the macromolecules are gradually released into the tear fluid by ionic dissociation. In the invention, various active ingredients such as ingredients for improving wearing feeling and anti-inflammatory ingredients for reducing inflammation can be appropriately employed as such an anionic compound and a cationic compound. Accordingly, the active ingredients are also gradually released onto the eyeball when it is worn on the eye, thereby continuously exerting the advantages of the active ingredients over a long period of time.

Further, in the SCL package product according to the invention, after the production thereof, the SCL is immersed in the predetermined liquid medium for a long period of time until it is shipped and opened by consumers (users). Accordingly, the anionic compound and the cationic compound contained in such a liquid medium sufficiently enter the inside of the matrix of the SCL to be ion bonded, thereby forming the macromolecules while being effectively entangled in the matrix tissue of the SCL. Thus, the advantages of the invention as described above can be more advantageously exerted.

In addition, also in the packaging solution for the SCL and the sustained-release SCL according to the invention, when the SCL using the predetermined liquid medium is worn on the eye, the advantage of improved wearing feeling as described above and the desired advantages due to the active ingredients are continuously obtained over a long period of time. Further, according to the method of shipping, the method of packaging and the method of treating the SCL of the invention, the excellent advantages as described above are advantageously obtained.

### BRIEF DESCRIPTION OF THE DRAWINGS

The above and other objects, features, advantages and technical and industrial significance of the present invention will be better understood by reading the following detailed description of a preferred embodiment of the invention, when considered in connection with the accompanying drawings, in which:
Fig. 1 is a vertical sectional illustrative view showing one embodiment of a SCL package product 2 of the invention;
Fig. 2 is an image view showing a contact lens at the time when an anionic compound having two or more anionic functional groups in one molecule and a cationic compound having two or more cationic functional groups in one molecule are allowed to be contained in a hydrated contact lens, and a state of those compounds;
Fig. 3 is graphs showing the results of a sustained-release test of SCL package products according to the invention; and
Fig. 4 is other graphs showing the results of a sustained-release test of SCL package products according to the invention.

### DETAILED DESCRIPTION OF THE INVENTION

To further clarify the present invention, there will be described a typical embodiment of the invention in detail with reference to the accompanying drawing.

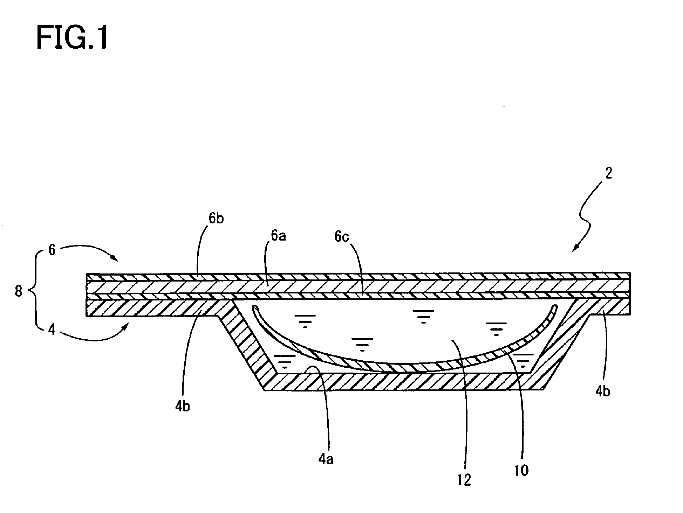

Referring first to Fig. 1, one example of a SCL package product according to the invention is schematically shown in a vertical cross sectional view. As apparent from this Fig. 1, a SCL package product 2 comprises a sealable packaging container 8 including a container main body 4 and a cover sheet 6, one SCL 10 housed in the packaging container 8, and a liquid medium 12 housed in the packaging container 8 together with this SCL 10 which is immersed in the liquid medium 12.

As shown in Fig. 1, the container main body 4 has a housing cavity 4a opened upward, and an opening peripheral portion of the housing cavity 4a is extended outward to form a flange portion 4b. The forming material of the container main body 4 is not particularly limited. It is generally formed by a plastic material, such as polypropylene, which is chemically stable to the liquid medium 12 which will be described later, and have elution resistance.

The cover sheet 6 has a laminated structure comprising a base sheet 6a and resin film layers 6b and 6c adhered to an upper side and a lower side of the base sheet 6a, respectively, with a known adhesive. Although the forming material of the base sheet 6a is not particularly limited, a metal foil, a plastic film, synthetic paper or the like is generally advantageously used. Among them, an aluminum foil is particularly preferably used. Further, although the forming material of the resin film layers 6b and 6c is also not limited, a synthetic resin such as polypropylene and polyethylene is generally preferably used, because it has sealing properties and can advantageously maintain an aseptic condition in the container. Then, such a cover sheet 6 is superposed on an upper surface of the flange portion 4b of the container main body 4 in such a manner that the cover sheet 6 fluid-tightly covers the opening of the housing cavity 4a formed in the above-mentioned container main body 4, and adhered by heat sealing or by using a known adhesive, thereby sealing the packaging container 8.

As for the SCL 10 housed in such a packaging container 8, the kind thereof is not particularly limited. Examples of the SCL include all of various known hydrated soft contact lenses such as HEMA (hydroxyethyl methacrylate)-based, N-VP (N-vinyl-2-pyrrolidone)-based, MAA (methacrylic acid)-based and PMMA (dimethylacrylamide)-based lenses, and silicone hydrogel-based soft contact lenses which have recently become popular.

In the invention, as the liquid medium housed in the packaging container 8 together with such a SCL 10 which is immersed in the liquid medium, the liquid medium 12 containing at least one anionic compound having two or more anionic functional groups in one molecule and at least one cationic compound having two or more cationic functional groups in one molecule is used in place of a liquid medium such as saline which has been conventionally used in order to merely prevent drying of a lens.

The anionic compound which can be contained in the liquid medium 12 according to the invention is not particularly limited. Any known compound of known ophthalmologically acceptable anionic compounds can be appropriately employed according to its purpose as long as it has two or more anionic functional groups in one molecule. In particular, in the invention, there is advantageously employed an anionic compound having at least one of a carboxyl group, a sulfo group (sulfonic acid group) and a phosphoric acid group as the anionic functional group. Specifically, there is advantageously used an anionic compound such as an anionic polysaccharide, glycyrrhizic acid, a salt of glycyrrhizic acid, a purine nucleotide or a peptide constituted by a plurality of acidic amino acids.

As the above-mentioned anionic polysaccharide, there is advantageously used one selected from the group consisting of chondroitin sulfuric acid, a salt of the chondroitin sulfuric acid, hyaluronic acid, a salt of the hyaluronic acid, alginic acid and a salt of the alginic acid. Specific examples thereof include chondroitin sulfuric acid, sodium chondroitin sulfuric acid, hyaluronic acid, sodium hyaluronate, alginic acid, and sodium alginate.

As the above-mentioned glycyrrhizic acid or salt thereof, specifically, there is advantageously used glycyrrhizic acid, disodium glycyrrhizinate, trisodium glycyrrhizinate, dipotassium glycyrrhizinate, monoammonium glycyrrhizinate or the like.

As the above-mentioned purine base nucleotide, there is preferably employed a phosphoric acid ester of adenosine such as adenosine monophosphate, adenosine diphosphate or adenosine triphosphate, a phosphoric acid ester of guanosine such as guanosine monophosphate, guanosine diphosphate or guanosine triphosphate, or the like.

In addition, as the above-mentioned peptide constituted by a plurality of acidic amino acids, specifically, polyglutamic acid or the like is preferably employed.

The cationic compound contained in the above-mentioned liquid medium 12 is not particularly limited. As long as it is a known ophthalmologically acceptable anionic compound and has two or more cationic functional groups in one molecule, any known compound may be appropriately employed according to its purpose. In particular, in the invention, it is preferable to employ one having at least one of an amino group, an ammonium group and a biguanide group.

As such a cationic compound, specifically, there is advantageously exemplified one selected from the group consisting of a vitamin, a purine base and a nucleoside derived therefrom. More specifically, examples of the vitamin include vitamin B₁, vitamin B₁₂, example of the purine base include adenine and guanine, and further, examples of the nucleoside derived from the purine base include adenosine and guanosine.

However, the anionic compound and the cationic compound to be used in the invention are not limited to the above examples. In addition to the compounds described above, there may be used, for example, dextran sulfuric acid, a salt thereof or the like as the anionic compound, and chlorpheniramine, a salt thereof, allantoin or the like as the cationic compound. Further, in the invention, such anionic compounds and cationic compounds may be each used either alone or as a combination of two or more thereof. Furthermore, the combination of the anionic compound and the cationic compound is not limited at all, and appropriately selected according to their purpose.

As described above, in the soft contact lens package product 2 according to the invention, as the liquid medium in which the SCL 10 is immersed, the liquid medium 12 containing at least one anionic compound having two or more anionic functional groups in one molecule and at least one cationic compound having two or more cationic functional groups in one molecule is used in place of the conventional liquid medium such as saline. Accordingly, the anionic compound and the cationic compound enter the matrix (tissue) of the SCL immersed in such liquid medium 12, and bind to each other by ionic interaction, independently of the hydrated polymer (hydrogel) constituting the SCL 10. Thus, the pseudo macromolecules are formed in the matrix of the SCL 10.

In the SCL 10 with such pseudo macromolecules formed in the lens tissue, such pseudo macromolecules are also present on the lens surface and are exposed to the outside, so that the viscosity of the liquid medium 12 on the lens surface is increased. Accordingly, friction between the SCL 10 and the cornea, which is caused when the SCL 10 is worn on the eye, is decreased, thereby improving wearing feeling of the SCL 10 to the eye. In the invention, the pseudo macromolecules having such a thickening effect are formed by bond caused by ionic interaction, and such macromolecules are formed while being entangled in the matrix tissue constituting the SCL 10. Accordingly, the above-mentioned macromolecules can be advantageously prevented from being immediately washed away with the tear fluid when the SCL 10 is worn on the eye, thereby extremely advantageously exerting improvement of wearing feeling by such pseudo macromolecules over a long period of time.

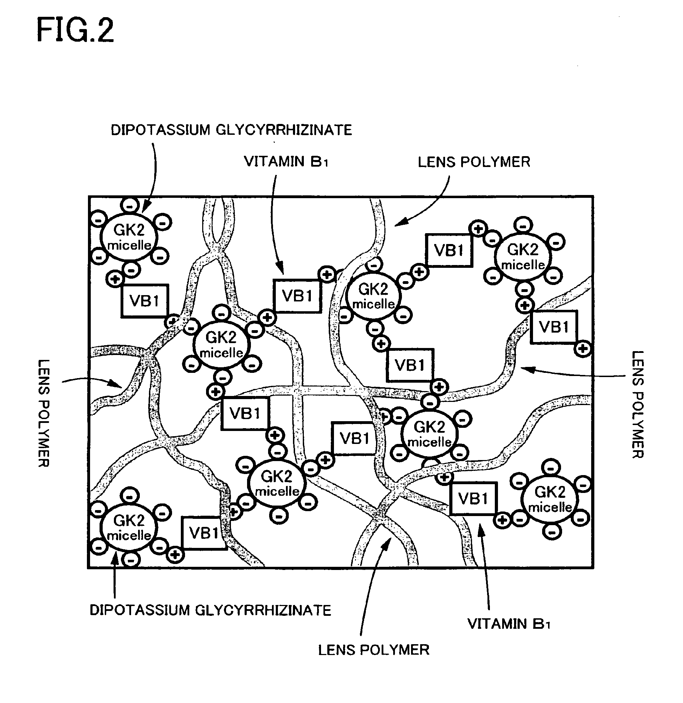

Specifically, for example, as shown in Fig. 2, when dipotassium glycyrrhizinate (GK2) is used as the anionic compound and vitamin B₁ (VB1) is used as the cationic compound, the dipotassium glycyrrhizinate forms micelles in an aqueous medium, and such dipotassium glycyrrhizinate micelles (GK2 micelles) and vitamin B₁ (VB1) form an ionic bond with each other in the inside of the matrix of the soft contact lens and on the surface thereof, while being entangled in polymer molecule chains constituting the SCL 10, thereby constituting the pseudo macromolecules so as to be restrained by the matrix tissue of the SCL 10.

By the way, in the SCL package product 2 according to the invention, the anionic compound and the cationic compound constituting the macromolecules are gradually released into the tear fluid from the above-mentioned macromolecules by ionic dissociation when the SCL 10 is worn on the eye. In the invention, the active ingredients which realize the desired advantages can be appropriately employed as those anionic compound and cationic compound according to their purpose. When such ingredients are employed, such active ingredients are gradually released when the SCL 10 is worn on the eye, thereby continuously exerting the advantages which is achieved by such ingredients over a long period of time.

Specifically, when the glycyrrhizic acid or a salt thereof as described above is used as the above-mentioned anionic compound, the ingredient exerts an anti-inflammatory effect in which inflammation of the eye is reduced by gradual release of the ingredient from the pseudo macromolecules when the SCL 10 is worn on the eye. Further, when the anionic polysaccharide such as chondroitin sulfuric acid as described above is used as the above-mentioned anionic compound, moisture retention or wettability of the SCL 10 is increased by sustained release of the ingredient, thereby imparting more excellent wearing feeling to the SCL 10. Furthermore, when the above-mentioned dextran sulfuric acid or a salt thereof is used, moisture retention or wettability of the SCL 10 can be advantageously increased by sustained release of such an ingredient.

Meanwhile, when vitamin B₁ is used as the above-mentioned cationic compound, asthenopia is healed by sustained release of such an ingredient. Further, when vitamin B₁₂ is used, a focus adjustment function of the ciliary muscle is improved. Furthermore, when the above-mentioned chlorpheniramine or salt thereof is used as the above-mentioned cationic compound, an anti-inflammatory effect or an antihistaminic effect is obtained by sustained release of such an ingredient. In addition, when the above-mentioned allantoin is used, an anti-inflammatory effect is continuously obtained over a long period of time.

In the invention, concentrations of such an anionic compound and a cationic compound in the liquid medium 12 are each not particularly limited, and appropriately determined according to the contact lens used, the kind of anionic compound and cationic compound, and the like. However, the concentrations of the anionic compound and the cationic compound are each generally about 0.005 to 0.5 w/w%, and preferably about 0.01 to 0.2 w/w%. Specifically, when dipotassium glycyrrhizinate is used as the anionic compound, the concentration of the compound is preferably 0.01 to 0.25 w/w%, and more preferably about 0.04 to 0.125 w/w%. Further, when vitamin B₁ or adenine is used as the cationic compound, the concentration of the compound is preferably 0.005 to 0.5 w/w%, and more preferably about 0.01 to 0.2 w/w%.

When the concentrations of the anionic compound and the cationic compound are too small, there may cause a risk of insufficient formation of the pseudo macromolecules in the matrix of the SCL 10 or on the surface thereof. Accordingly, it might become difficult to sufficiently exert the above-mentioned advantages of the invention. On the other hand, when the concentrations of the anionic compound and the cationic compound are too large, the viscosity is excessively increased, which may cause problems such as a decrease in handling properties, deterioration of wearing feeling and a poor field of view.

Further, in the invention, when one of the anionic compound and the cationic compound has excessive charges, the compound having excessive charges is not taken into the pseudo macromolecules by the ion bond, and thus it exists independently. Accordingly, the compound having excessive charges is immediately washed away on the eyeball when the SCL 10 is worn on the eye, so that it might become difficult to sufficiently exert the above-mentioned advantages of the invention. It is therefore desirable that the concentrations of the anionic compound and the cationic compound are adjusted to electrically equivalent ratios.

Further, in the liquid medium 12 used in the SCL package product 2 according to the invention, various additives such as a chelating agent, a tonicity adjusting agent, a nonionic surfactant, a polysaccharide compound and the like, which can be used in the liquid media for the conventional SCL package products, are appropriately incorporated at a concentration similar to the conventional one.

The above-mentioned chelating agent is incorporated herein in order to prevent deposition or adsorption of calcium and the like contained in the aqueous medium included in the liquid medium 12 and the like, to the SCL 10. Examples of the chelating agent include ethylenediaminetetraacetic acid (EDTA), disodium ethylenediaminetetraacetate (EDTA·2Na), trisodium ethylenediaminetetraacetate (EDTA·3Na). Such a chelating agent is advantageously used generally at a concentration of about 0.01 to 0.5 w/w%.

Further, above-mentioned tonicity adjusting agent is incorporated in order to adjust the osmotic pressure of the liquid medium 12 close to the osmotic pressure of the tear fluid, thereby decreasing stimulation to the eye when the SCL 10 is worn on the eye. Generally, an inorganic salt such as sodium chloride or potassium chloride, or a nonionic tonicity adjusting agent such as a saccharide, a sugar alcohol, a polyhydric alcohol such as glycerol, propylene glycol or polyethylene glycol, an ether thereof or an ester thereof is used at an appropriate concentration. For example, the inorganic salt is used at a concentration of 0 to 1.2 w/w%, preferably about 0 to 0.9 w/w%. The nonionic tonicity adjusting agent is used at a concentration of about 0 to 1 w/w%. In the invention, it is desirable that the osmotic pressure of the liquid medium 12 is adjusted to the range of about 0.75 to 1.6 and preferably to the range of about 0.8 to 1.5 as the osmotic pressure ratio to saline (taking the osmotic pressure of saline as 1).

Furthermore, the above-mentioned nonionic surfactant is incorporated in order to prevent adhesion of stains such as proteins and lipids to the SCL 10. Examples of the nonionic surfactant include a polyoxyethylene-polyoxypropylene block copolymer or a derivative thereof, a polyethylene glycol derivative such as a polyoxyethylene alkyl phenyl ether-formaldehyde condensate such as tyloxapol, a polyoxyethylene sorbitan fatty acid ester (Polysolvate) such as polyoxyethylene sorbitan monooleate (Polysolvate 80), a sorbitan fatty acid ester such as sorbitan sesquioleate, a glycerin fatty acid ester such as glyceryl monostearate, a polyethylene glycol fatty acid ester such as polyethylene glycol monostearate, a polyoxyethylene alkyl ether such as polyoxyethylene lauryl ether, polyoxyethylene hydrogenated caster oil, polyoxyethylene caster oil, a sucrose fatty acid ester, a polyoxyethylene alkyl ether carboxylic acid or an ester thereof. The nonionic surfactant is advantageously used, for example, at a concentration of about 0.001 to 5 w/w%, preferably about 0.005 to 2 w/w%.

In addition, the above-mentioned polysaccharide compound is incorporated in order to adjust the viscosity of the liquid medium 12. Examples of the polysaccharide compound include cellulose, starch, dextrin, dextran, pullulan and a derivative thereof. The polysaccharide compound is advantageously used generally at a concentration of about 0.002 to 3.0 w/w%.

Then, as the aqueous medium to which the chelating agent, the tonicity adjusting agent, the nonionic surfactant, the polysaccharide compound and the like are incorporated as needed in addition to the anionic compound and the cationic compound as described above to obtain the desired liquid medium 12, any solution mainly composed of water may be used as well as water itself such as tap water or purified water, as long as it has high safety to living bodies and is ophthalmologically sufficiently acceptable.

In the invention, the above-described liquid medium 12 and the SCL 10 are housed in the above-mentioned packaging container 8, and the packaging container 8 is sealed, thereby constituting the desired SCL package product 2. Then, the inside of such a packaging container 8 is subjected to treatment such as heat sterilizing treatment as needed, thereby obtaining a sterile state. Such a sterile state is important on providing the SCL 10 to SCL wearers in an aseptic condition.

As described above, in the SCL package product 2 according to the invention, the package product 8 is sealed and the inside of the package product 8 is in the sterile state, so that the development of microorganisms such as bacteria and fungi can be effectively prevented in the packaging container 8 from when the SCL package product 2 is produced until when it is opened. Accordingly, the SCL 10 housed in the packaging container 8 can be advantageously sanitized.

By the way, the SCL package product 2 according to the invention as described above can be advantageously obtained desirably by packaging the SCL 10 in the following manner.

Specifically, first, the container main body 4 and the cover sheet 6 constituting the package product 8, which has the structure as described above, are each produced and prepared according to the well-known resin molding operation or sheet laminating operation, which is the conventionally known method of producing a SCL packaging container.

Meanwhile, the anionic compound and the cationic compound as described above are added to the predetermined aqueous medium such as purified water at a predetermined ratio together with the hydroxyl group-containing nonionic compound and various other additives to be contained therein, thereby preparing the liquid medium 12.

Then, the liquid medium 12 prepared above is housed in the housing cavity 4a of the container main body 4 previously prepared, together with the one SCL 10, and in the state where the SCL 10 is immersed in such a liquid medium 12, the packaging container 8 is sealed by adhesion of the cover sheet 6 and the flange portion 4b of the container main body 4 with the adhesive or the like, thereby producing the desired SCL package product 2.

In the invention, it is desirable that heating treatment using an autoclave or the like is further performed to such a sealed packaging container 8. In the heating treatment using an autoclave or the like, conditions are appropriately determined so as not to adversely affect the SCL 10 housed in the packaging container 8. In general, the heating treatment is performed at about 120 to 125°C and for about 10 to 30 minutes.

The heating treatment makes the sterile state in the packaging container 8 more sure, and the ion bond already formed between the anionic compound and the cationic compound is once cleaved in the prepared liquid medium 12. Accordingly, the anionic compound and the cationic compound more sufficiently enter the matrix of the SCL 10. Further, the anionic compound and the cationic compound allowed to sufficiently enter the matrix of the SCL 10 as described above form an ion bond again while being entangled in polymer molecule chains constituting the SCL 10, with slowly cooling the liquid medium 12, thereby advantageously obtaining the pseudo macromolecules integrated with the matrix of the SCL 10. Thus, the above-mentioned advantages of the invention can be more effectively exerted.

The SCL package product 2 according to the invention is obtained as described above. The SCL package product 2 thus obtained is shipped after the production thereof, and sent to a SCL wearer through a distribution process, like general SCL package products.

In the SCL package product 2 according to the invention thus shipped, the anionic compound and the cationic compound sufficiently enter the matrix of the SCL 10 over a long period of time from when the SCL package product 2 is produced until when it is shipped and opened by the SCL wearer as the user, so that the above-mentioned macromolecules are integrally formed more advantageously inside and outside the matrix of the SCL 10. Thus, the above-mentioned advantages of the invention can be extremely advantageously enjoyed.

Further, the invention is also directed to a packaging solution for a SCL comprising the liquid medium 12 as described above, a method of treating a SCL comprising the steps of immersing the SCL 10 in the liquid medium 12, and ion bonding the anionic compound and the cationic compound in the matrix of the SCL 10 to constitute pseudo macromolecules, and further, a sustained-release SCL in which the anionic compound and the cationic compound are gradually released from the macromolecules formed in the matrix of the SCL 10 to the tear fluid when the SCL 10 is worn on the eye. Also in the packaging solution, the method of treating a SCL and the sustained-release SCL, the above-mentioned advantage of the invention can be advantageously obtained.

While the preferred embodiment of the invention has been described in detail, for illustrative purpose only, it is to be understood that the present invention is not limited to the details of the illustrated embodiment.

For instance, in the above-mentioned embodiments, only one SCL 10 has been housed in the packaging container 8. However, in the invention, the number of such SCLs 10 housed is not particularly limited as long as at least one SCL 10 is housed.

Further, the packaging container 8 is also not limited at all to one having the structure shown in Fig. 1. Any various conventionally known contact lens packaging containers may be appropriately employed according to their purpose. For example, the contact lens packaging containers as disclosed in WO 2006/061886 and WO 2004/019114 may be appropriately employed.

In the above-mentioned embodiments, the heating treatment is performed to the sealed packaging container 8, thereby concurrently performing heat sterilizing treatment to put the inside of the packaging container 8 into the sterile state. However, when such heating treatment is not performed, the inside of the packaging container 8 is appropriately put into the sterile state separately by other known sterilizing treatment such as sterilizing treatment using ultraviolet rays or sterilizing treatment with a disinfectant. Further, in the above-mentioned embodiments, the sterilizing treatment is performed after the packaging container 8 has been sealed to put the inside of the packaging container 8 into the sterile state. However, it is also possible to perform the heating treatment (heat sterilizing treatment) by the autoclave or the sterilizing treatment by ultraviolet irradiation before the packaging container 8 is sealed, as long as the aseptic condition is kept until the sealing process of the packaging container 8 is performed.

It is to be understood that the present invention may be embodied with various changes and modifications which may occur to those skilled in the art, without departing from the spirit and scope of the invention defined in the attached claims.

### EXAMPLES

To further clarify the present invention, some examples of the invention will be described. It is to be understood that the invention is not limited to the details of illustrated examples and the forgoing description, but may be embodied with various changes, modifications, and improvements, which may occur to those skilled in the art without departing from the scope of the invention.

### -Test Solution 1-

Dipotassium glycyrrhizinate (manufactured by MARUZEN PHARMACEUTICALS CO., LTD.) was provided as the anionic compound having two or more anionic functional groups in one molecule, and vitamin B₁ (thiamine hydrochloride, manufactured by BASF Japan Ltd.) was provided as the cationic compound having two or more cationic functional groups in one molecule. Further, ethylenediaminetetraacetic acid (EDTA) was provided as the chelating agent, and furthermore, sodium chloride was provided as the tonicity adjusting agent. Then, those were each added to purified water at concentrations shown in the following TABLE 1, and mixed to prepare test solution 1. Further, the viscosity (cP) at 20°C of test solution 1 was measured by using a small-sized vibro viscometer CJV-5000 (manufactured by A & D Company, Limited.). The results thereof are shown together in the following TABLE 1.

### -Test Solution 2-

Test solution 2 was prepared in the same manner as in test solution 1 with the exception that adenine (manufactured by Wako Pure Chemical Industries, Ltd.) was used as the cationic compound having two or more cationic functional groups in one molecule in place of vitamin B₁ described above. Further, the viscosity of test solution 2 was measured in the same manner as in test solution 1. The results thereof are shown together in the following TABLE 1.

### -Comparative Solution 1-

Comparative solution 1 was prepared in the same manner as in test solution 1 with the exception that the cationic compound having two or more cationic functional groups in one molecule was not used. Further, the viscosity of such comparative solution 1 was measured in the same manner as in test solution 1. The results thereof are shown together in the following TABLE 1.

### -Comparative Solution 2-

Comparative solution 2 was prepared in the same manner as in test solution 1 with the exception that the anionic compound having two or more anionic functional groups in one molecule was not used. Further, the viscosity of comparative solution 2 was measured in the same manner as in test solution 1. The results thereof are shown together in the following TABLE 1.

### -Comparative Solution 3-

Comparative solution 3 was prepared in the same manner as in test solution 2 with the exception that the anionic compound having two or more anionic functional groups in one molecule was not used. Further, the viscosity of comparative solution 3 was measured in the same manner as in test solution 1. The results thereof are shown together in the following TABLE 1.

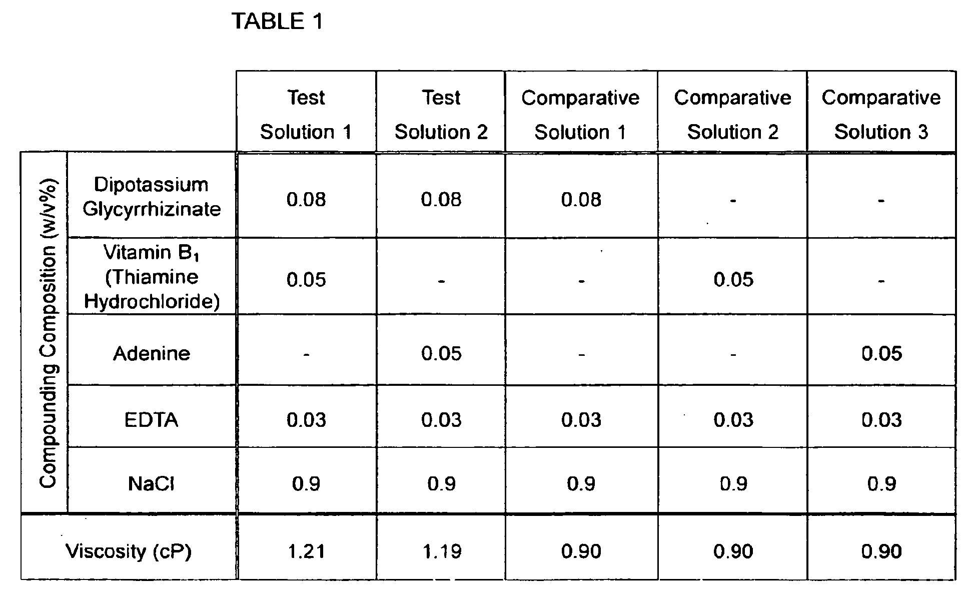

As apparent from the results of TABLE 1, it is observed that test solution 1 and test solution 2 containing the cationic compound having two or more cationic functional groups in one molecule together with the anionic compound having two or more anionic functional groups in one molecule have high viscosity (viscous property) compared to comparative solutions 1 to 3 containing only either one of such an anionic compound and a cationic compound.

### -Test Solution 3-

Dipotassium glycyrrhizinate (GK2, manufactured by MARUZEN PHARMACEUTICALS CO., LTD.) was provided as the anionic compound having two or more anionic functional groups in one molecule, and vitamin B₁ (thiamine hydrochloride, manufactured by BASF Japan Ltd.) was provided as the cationic compound having two or more cationic functional groups in one molecule. Then, those were each added to a citric acid buffer of pH 5 at concentrations shown in the following TABLE 2, and mixed to prepare test solution 3.

Further, Menicon TWO-WEEK Premio (manufactured by MENICON CO., LTD.) was provided as soft contact lens a, Menicon Month Wear (manufactured by MENICON CO., LTD.) was provided as soft contact lens b and Menicon Soft MA (manufactured by MENICON CO., LTD.) was provided as soft contact lens c, and the soft contact lenses thus provided were each immersed in 2 mL of test solution 3, and subjected to an autoclave treatment at 120°C for 20 minutes.

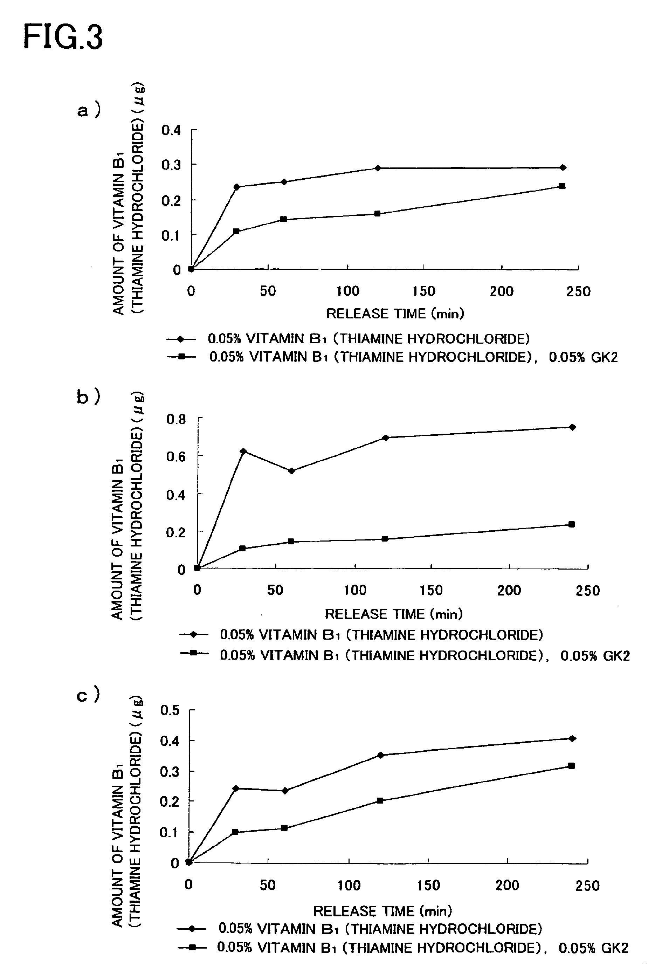

Thereafter, the soft contact lenses were each taken out of test solution 3, and lightly rinsed with a citric acid buffer of pH 5. Then, the soft contact lenses were each immersed in 2 mL of a citric acid buffer of pH 5. After elapses of 0 minutes, 30 minutes, 1 hour, 2 hours and 4 hours after immersion, a part of the citric acid buffer in which each soft contact lens had been immersed was taken out, and the solution taken out was appropriately diluted with distilled water, thereby preparing a solution for measurement. To 1 mL of such a solution for measurement, 150 µL of an aqueous sodium hydroxide solution having a concentration of 1 mol/L was added, and then, 150 µL of an aqueous potassium hexacyanoferrate (III) having a concentration of 1 w/v% was added. Then, the temperature was kept at 70°C for 30 minutes. For the resulting solution, fluorescence measurement was performed under the conditions of an excitation wavelength of 360 nm and an emission wavelength of 450 nm, thereby calculating the amount of vitamin B₁ released. The results obtained are shown together in TABLE 2 and Fig. 3.

### -Comparative Solution 4-

Comparative solution 4 was prepared in the same manner as in the above-mentioned test solution 3 with the exception that no dipotassium glycyrrhizinate was used. The three kinds of soft contact lenses were each immersed in comparative solution 4 in the same manner as described above, and then, immersed in a citric acid buffer. The amount of vitamin B₁ released in the citric acid buffer was calculated by performing the fluorescence measurement. The results obtained are shown together in TABLE 2 and Fig. 3.

### -Test Solution 4-

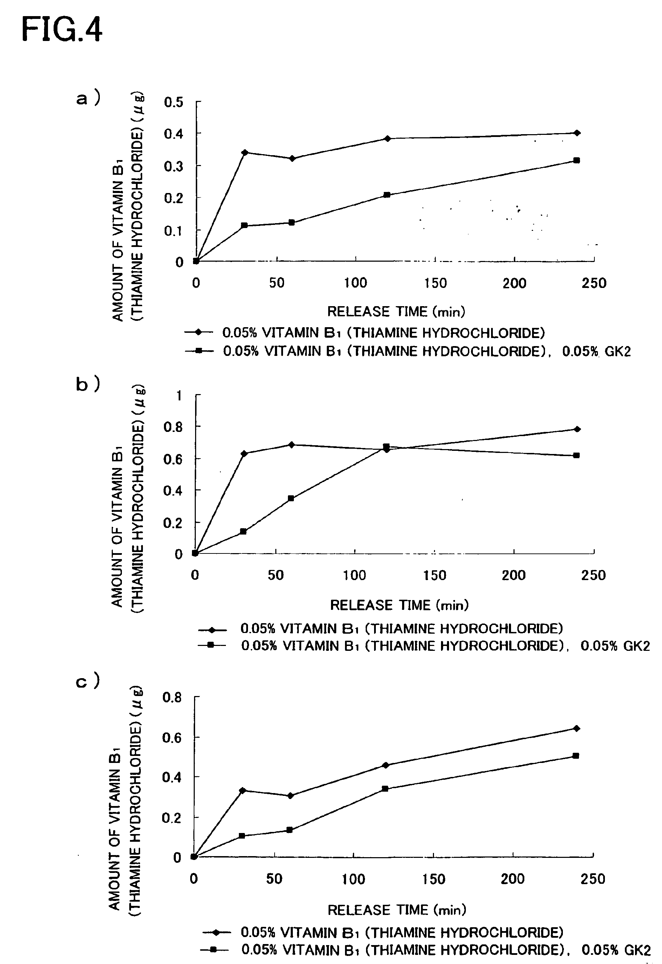

Test solution 4 was prepared in the same manner as in the above-mentioned test solution 3 with the exception that the pH of the citric acid buffer was 7. The three kinds of soft contact lenses were each immersed in test solution 4 in the same manner as described above, and then, immersed in a citric acid buffer. The amount of vitamin B₁ released in the citric acid buffer was calculated by performing the fluorescence measurement. The results obtained are shown together in TABLE 2 and Fig. 4.

### -Comparative Solution 5-

Comparative solution 5 was prepared in the same manner as in the above-mentioned test solution 4 with the exception that no dipotassium glycyrrhizinate was used. The three kinds of soft contact lenses were each immersed in comparative solution 5 in the same manner as described above, and then, immersed in a citric acid buffer. The amount of vitamin B₁ released in the citric acid buffer was calculated by performing the fluorescence measurement. The results obtained are shown together in TABLE 2 and Fig. 4.

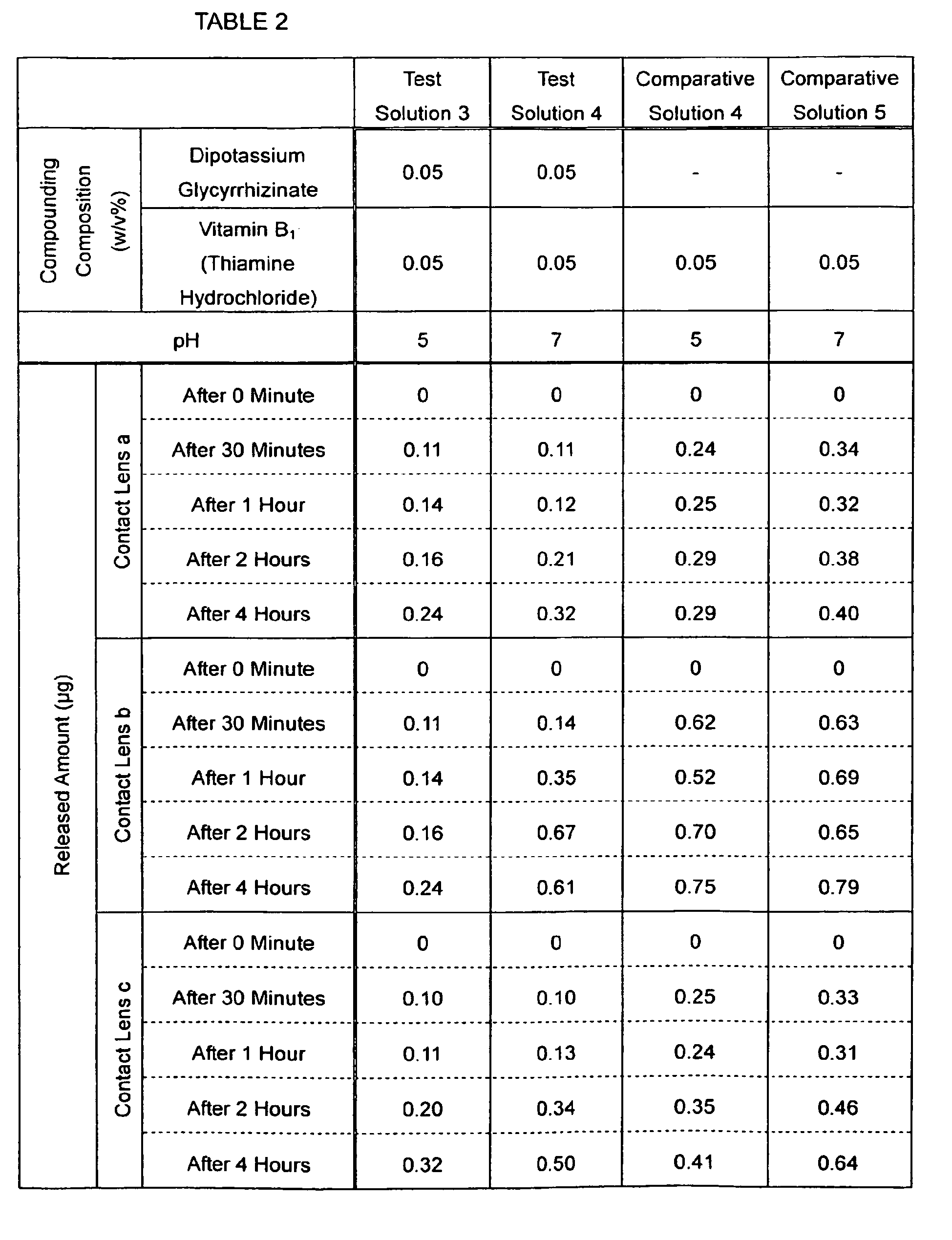

As apparent from the results shown in TABLE 2, Fig. 3 and Fig. 4, it was observed that the cationic compound was gradually released over a longer period of time in test solution 3 and test solution 4 containing the cationic compound having two or more cationic functional groups in one molecule together with the anionic compound having two or more anionic functional groups in one molecule, compared to comparative solutions 4 and 5 containing only the cationic compound. Further, in comparative solution 4 and comparative solution 5 containing only the cationic compound having two or more cationic functional groups in one molecule and not containing the anionic compound having two or more anionic functional groups in one molecule, it was observed that the cationic compound was released in large amounts for first 30 minutes after immersion in the buffer, and thereafter, came to be scarcely released.

## Claims

1. A soft contact lens package product (2), comprising:
a liquid medium (12) containing at least one anionic compound having two or more anionic functional groups in one molecule and at least one cationic compound having two or more cationic functional groups in one molecule;
at least one hydrated soft contact lens (10) immersed in the liquid medium; and
a sealable container (8) in which the liquid medium and the at least one hydrated soft contact lens are housed, the sealable container being sealed so that the inside thereof being held in a sterile condition.

2. The soft contact lens package product according to claim 1, wherein the anionic compound has at least one of a carboxyl group, a sulfo group and a phosphoric acid group as the anionic functional group.

3. The soft contact lens package product according to claim 1 or 2, wherein the anionic compound is selected from the group consisting of an anionic polysaccharide, glycyrrhizic acid, a salt of the glycyrrhizic acid, a purine base nucleotide and a peptide.

4. The soft contact lens package product according to claim 3, wherein the anionic polysaccharide is selected from the group consisting of chondroitin sulfuric acid, a salt of the chondroitin sulfuric acid, hyaluronic acid, a salt of the hyaluronic acid, alginic acid and a salt of the alginic acid.

5. The soft contact lens package product according to any one of claims 1 to 4, wherein the cationic compound has at least one of an amino group, an ammonium group and a biguanide group as the cationic functional group.

6. The soft contact lens package product according to any one of claims 1 to 5, wherein the cationic compound is selected from the group consisting of a vitamin, a purine base, a nucleoside derived from the purine base, chlorpheniramine, a salt of the chlorpheniramine, and allantoin.

7. The soft contact lens package product according to claim 6, wherein the vitamin is selected from the group consisting of vitamin B₁ and vitamin B₁₂.

8. The soft contact lens package product according to claim 6 or 7, wherein the purine base is one of adenine and guanine, and the nucleoside derived from the purine base is one of adenosine and guanosine.

9. A method of shipping a soft contact lens, comprising the step of shipping at least one hydrated soft contact lens in the form of the soft contact lens package product according to any one of claims 1 to 8, thereby providing the at least one hydrated soft contact lens to a wearer thereof.

10. A method of packaging a soft contact lens, comprising the steps of:
providing a sealable container (8) having a size sufficient to house at least one hydrated soft contact lens (10);
providing a liquid medium (12) to be housed in the container, the liquid medium containing at least one anionic compound having two or more anionic functional groups in one molecule and at least one cationic compound having two or more cationic functional groups in one molecule;
housing the liquid medium in the container together with the at least one hydrated soft contact lens; and
sealing the container in a state where the at least one hydrated contact lens is immersed in the liquid medium.

11. The method of packaging a soft contact lens according to claim 10, further comprising the step of heat treating the sealed container.

12. A packaging solution for a hydrated soft contact lens, comprising a liquid medium containing at least one anionic compound having two or more anionic functional groups in one molecule and at least one cationic compound having two or more cationic functional groups in one molecule.

13. A method of treating a soft contact lens, comprising the step of immersing a hydrated soft contact lens (10) in a liquid medium (12) containing at least one anionic compound having two or more anionic functional groups in one molecule and at least one cationic compound having two or more cationic functional groups in one molecule, the at least one anionic compound and the at least one cationic compound being allowed to enter a matrix of the hydrated soft contact lens and be ion bonded in the matrix to constitute pseudo macromolecules.

14. The method of treating a soft contact lens according to claim 13, wherein the liquid medium in which the hydrated soft contact lens is immersed is heated.

15. A sustained-release soft contact lens in which at least one anionic compound having two or more anionic functional groups in one molecule and at least one cationic compound having two or more cationic functional groups in one molecule are allowed to enter a matrix of a hydrated soft contact lens (10) and are ion bonded in the matrix thereof to constitute pseudo macromolecules, the compounds being gradually released from the macromolecules to the tear fluid when the contact lens is worn on an eye.

## Patentansprüche

1. Verpackungsprodukt (2) für weiche Kontaktlinsen, das Folgendes umfasst:
ein flüssiges Medium (12), das zumindest eine anionische Verbindung mit zwei oder mehr funktionellen anionischen Gruppen in einem Molekül und zumindest eine kationische Verbindung mit zwei oder mehr funktionellen kationischen Gruppen in einem Molekül enthält;
zumindest eine hydratisierte weiche Kontaktlinse (10), die in das flüssige Medium eingetaucht ist; und
einen verschließbaren Behälter (8), in dem das flüssige Medium und die zumindest eine hydratisierte weiche Kontaktlinse aufgenommen sind, wobei der verschließbare Behälter verschlossen ist, sodass sein Inneres in sterilem Zustand gehalten wird.

2. Verpackungsprodukt für weiche Kontaktlinsen nach Anspruch 1, worin die anionische Verbindung als funktionelle anionische Gruppe zumindest eine von einer Carboxylgruppe, Sulfogruppe und Phosphorsäuregruppe aufweist.

3. Verpackungsprodukt für weiche Kontaktlinsen nach Anspruch 1 oder 2, worin die anionische Verbindung aus der aus einem anionischen Polysaccharid, Glycyrrhizinsäure, einem Salz der Glycyrrhizinsäure, einem Purinbasen-Nucleotid und einem Peptid bestehenden Gruppe ausgewählt ist.

4. Verpackungsprodukt für weiche Kontaktlinsen nach Anspruch 3, worin das anionische Polysaccharid aus der aus Chondroitinschwefelsäure, einem Salz der Chondroitinschwefelsäure, Hyaluronsäure, einem Salz der Hyaluronsäure, Alginsäure und einem Salz der Alginsäure bestehenden Gruppe ausgewählt ist.

5. Verpackungsprodukt für weiche Kontaktlinsen nach einem der Ansprüche 1 bis 4, worin die kationische Verbindung als funktionelle kationische Gruppe zumindest eine von einer Aminogruppe, Ammoniumgruppe und Biguanidgruppe aufweist.

6. Verpackungsprodukt für weiche Kontaktlinsen nach einem der Ansprüche 1 bis 5, worin die kationische Verbindung aus der aus einem Vitamin, einer Purinbase, einem von der Purinbase abgeleiteten Nucleosid, Chlorpheniramin, einem Salz von Chlorpheniramin und Allantoin bestehenden Gruppe ausgewählt ist.

7. Verpackungsprodukt für weiche Kontaktlinsen nach Anspruch 6, worin das Vitamin aus der aus Vitamin B₁ und Vitamin B₁₂ bestehenden Gruppe ausgewählt ist.

8. Verpackungsprodukt für weiche Kontaktlinsen nach Anspruch 6 oder 7, worin die Purinbase aus Adenin und Guanin ausgewählt ist und das von der Purinbase abgeleitete Nucleosid Adenosin oder Guanosin ist.

9. Verfahren zum Versand einer weichen Kontaktlinse, das den Schritt des Versands zumindest einer hydratisierten weichen Kontaktlinse in Form des Verpackungsprodukts für weiche Kontaktlinsen nach einem der Ansprüche 1 bis 8 umfasst, wodurch die zumindest eine hydratisierte weiche Kontaktlinse einem Kontaktlinsenträger bereitgestellt wird.

10. Verfahren zur Verpackung einer weichen Kontaktlinse, das folgende Schritte umfasst:
die Bereitstellung eines verschließbaren Behälters (8), der ausreichend groß ist, um zumindest eine hydratisierte weiche Kontaktlinse (10) aufzunehmen;
die Bereitstellung eines flüssigen Mediums (12) zur Aufnahme in dem Behälter, wobei das flüssige Medium zumindest eine anionische Verbindung mit zwei oder mehr funktionellen anionischen Gruppen in einem Molekül und zumindest eine kationische Verbindung mit zwei oder mehr funktionellen kationischen Gruppen in einem Molekül enthält;
das Aufnehmen des flüssigen Mediums in dem Behälter gemeinsam mit der zumindest einen hydratisierten weichen Kontaktlinse und
das Verschließen des Behälters in einem Zustand, in dem die zumindest eine hydratisierte weiche Kontaktlinse in dem flüssigen Medium eingetaucht ist.

11. Verfahren zur Verpackung einer weichen Kontaktlinse nach Anspruch 10, das weiters den Schritt der Wärmebehandlung des verschlossenen Behälters umfasst.

12. Verpackungslösung für eine hydratisierte weiche Kontaktlinse, die ein flüssiges Medium umfasst, das zumindest eine anionische Verbindung mit zwei oder mehr funktionellen anionischen Gruppen in einem Molekül und zumindest eine kationische Verbindung mit zwei oder mehr funktionellen kationischen Gruppen in einem Molekül enthält.

13. Verfahren zur Behandlung einer weichen Kontaktlinse, das das Eintauchen einer hydratisierten weichen Kontaktlinse (10) in ein flüssiges Medium (12) umfasst, das zumindest eine anionische Verbindung mit zwei oder mehr funktionellen anionischen Gruppen in einem Molekül und zumindest eine kationische Verbindung mit zwei oder mehr funktionellen kationischen Gruppen in einem Molekül enthält, wobei ermöglicht wird, dass die zumindest eine anionische Verbindung und die zumindest eine kationische Verbindung in eine Matrix der hydratisierten weichen Kontaktlinse eindringen und in der Matrix über Ionenbindung gebunden werden, um Pseudomakromoleküle zu bilden.

14. Verfahren zur Behandlung einer weichen Kontaktlinse nach Anspruch 13, worin das flüssige Medium, in das die hydratisierte weiche Kontaktlinse eingetaucht wird, erhitzt wird.

15. Weiche Kontaktlinse mit verzögerter Freisetzung, in der ermöglicht wird, dass zumindest eine anionische Verbindung mit zwei oder mehr funktionellen anionischen Gruppen in einem Molekül und zumindest eine kationische Verbindung mit zwei oder mehr funktionellen kationischen Gruppen in einem Molekül in eine Matrix der hydratisierten weichen Kontaktlinse (10) eindringen und in der Matrix über Ionenbindung gebunden werden, um Pseudomakromoleküle zu bilden, wobei die Verbindungen von den Makromolekülen beim Tragen der Kontaktlinse auf dem Auge allmählich in die Tränenflüssigkeit freigesetzt werden.

## Revendications

1. Produit de conditionnement de lentilles de contact souples (2), comprenant:
un milieu liquide (12) contenant au moins un composé anionique ayant deux ou plusieurs groupes fonctionnels anioniques dans une molécule et au moins un composé cationique ayant deux ou plusieurs groupes fonctionnels cationiques dans une molécule;
au moins une lentille de contact souple hydratée (10) immergée dans le milieu liquide; et
un contenant (8) pouvant être rendu étanche dans lequel le milieu liquide et la au moins une lentille de contact souple hydratée sont logés le contenant apte à être scellé étant scellé de sorte que l'intérieur de celui-ci est maintenu dans un état stérile.

2. Produit de conditionnement de lentilles de contact souples selon la revendication 1, où le composé anionique possède au moins un d'un groupe carboxyle, d'un groupe sulfo et d'un groupe d'acide phosphorique comme groupe fonctionnel anionique.

3. Produit de conditionnement de lentilles de contact souples selon la revendication 1 ou 2, où le composé anionique est sélectionné dans le groupe consistant en polysaccharide anionique, acide glycyrrhizique, un sel de l'acide glycyrrhizique, un nucléotide à base de purine et un peptide.

4. Produit de conditionnement de lentilles de contact souples selon la revendication 3, où le polysaccharide anionique est sélectionné dans le groupe consistant en acide chondroitine sulfurique, un sel de l'acide chondroitine sulfurique, acide hyaluronique, un sel de l'acide hyaluronique, acide alginique et un sel de l'acide alginique.

5. Produit de conditionnement de lentilles de contact souples selon l'une quelconque des revendications 1 à 4, où le composé cationique possède au moins un d'un groupe aminé, d'un groupe ammonium et d'un groupe biguanide comme groupe fonctionnel cationique.

6. Produit de conditionnement de lentilles de contact souples selon l'une quelconque des revendications 1 à 5, où le composé cationique est sélectionné dans le groupe consistant en une vitamine, une base de purine, un nucléoside dérivé de la base de purine, chlorphéniramine, un sel de chlorphéniramine et allantoïne.

7. Produit de conditionnement de lentilles de contact souples selon la revendication 6, où la vitamine est sélectionnée dans le groupe consistant en vitamine B₁ et Vitamine B₁₂.

8. Produit de conditionnement de lentilles de contact souples selon la revendication 6 ou 7, où la base de purine est une d'adénine et de guanine, et le nucléoside dérivé de la base de purine est un parmi l'adénosine et la guanosine.

9. Procédé d'expédition d'une lentille de contact souple, comprenant l'étape consistant à expédier au moins une lentille de contact souple hydratée sous la forme d'un produit de conditionnement de lentille de contact souple selon l'une quelconque des revendications 1 à 8, en fournissant ainsi la au moins une lentille de contact souple hydratée à une personne qui la porte.

10. Procédé de conditionnement d'une lentille de contact souple, comprenant les étapes de:
réaliser un contenant (8) pouvant être rendu étanche d'une taille suffisante pour loger au moins une lentille de contact souple hydratée (10);
fournir un milieu liquide (12) à loger dans le contenant, le milieu liquide contenant au moins un composé anionique ayant deux ou plusieurs groupes fonctionnels anioniques dans une molécule et au moins un composé cationique ayant deux ou plusieurs groupes fonctionnels cationiques dans une molécule;
renfermer le milieu liquide dans le contenant ensemble avec la au moins une lentille de contact souple hydratée; et
rendre étanche le contenant dans un état où la au moins une lentille de contact hydratée est immergée dans le milieu liquide.

11. Procédé de conditionnement d'une lentille de contact souple selon la revendication 10, comprenant en outre l'étape d'un traitement thermique du contenant scellé.

12. Solution de conditionnement pour une lentille de contact souple hydratée, comprenant un milieu liquide contenant au moins un composé anionique ayant deux ou plusieurs groupes fonctionnels anioniques dans une molécule et au moins un composé cationique ayant deux ou plusieurs groupes fonctionnels cationiques dans une molécule.

13. Procédé de traitement d'une lentille de contact souple, comprenant l'étape consistant à immerger une lentille de contact souple hydratée (10) dans un milieu liquide (12) contenant au moins un composant anionique ayant deux ou plusieurs groupes fonctionnels anioniques dans une molécule et au moins un composé cationique ayant deux ou plusieurs groupes fonctionnels cationiques dans une molécule, le au moins un composé anionique et le au moins un composé cationique étant amenés à entrer dans une matrice de la lentille de contact souple hydratée et à être liés par ions dans la matrice pour constituer des pseudo-macromolécules.

14. Procédé de traitement d'une lentille de contact souple selon la revendication 13, où le milieu liquide dans lequel la lentille de contact souple hydratée est immergée, est chauffé.

15. Lentille de contact souple à libération prolongée, où au moins un composé anionique ayant deux ou plusieurs groupes fonctionnels anioniques dans une molécule et au moins un composé cationique ayant deux ou plusieurs groupes fonctionnels cationiques dans une molécule sont amenés à entrer dans une matrice d'une lentille de contact souple hydratée (10) et sont liés par ions dans la matrice de celle-ci pour constituer des pseudos-macromolécules, les composés étant progressivement libérés des macromolécules dans le liquide lacrymal lorsque la lentille de contact est portée sur un oeil.
